(19) Europäisches Patentamt
European Patent Office
Office européen des brevets

(11) EP 1 840 562 B1

(12) EUROPEAN PATENT SPECIFICATION

(45) Date of publication and mention of the grant of the patent:
**13.02.2019 Bulletin 2019/07**

(51) Int Cl.:
***G01N 27/414*** *(2006.01)*

(21) Application number: **06125701.0**

(22) Date of filing: **08.12.2006**

# (54) Method and apparatus for simultaneously detecting length and concentration of nucleic acid

Verfahren und Vorrichtung zur simultanen Detektion von Länge und Konzentration von Nucleinsäuren

Procédé et appareil de détection simultanée de la et de la longuer et concentration de acide nucléique

(84) Designated Contracting States:
**DE GB**

(30) Priority: **31.03.2006 KR 20060029807**

(43) Date of publication of application:
**03.10.2007 Bulletin 2007/40**

(73) Proprietor: **Samsung Electronics Co., Ltd.**
**Suwon-si, Gyeonggi-do, 443-742 (KR)**

(72) Inventors:
- **Kyu-sang, Lee**
**c/o Samsung Advanced Institute of Te**
**Gyeonggi-do (KR)**
- **Yeon-ja, Cho**
**c/o Samsung Advanced Institute of Tec**
**Gyeonggi-do (KR)**
- **Won-seok, Chung**
**c/o Samsung Adv. Institute of Tech**
**Gyeonggi-do (KR)**
- **Jeo-young, Shim**
**c/o Samsung Adv. Institute of Tec**
**Gyeonggi-do (KR)**
- **Kyu-tae, Yoo**
**c/o Samsung Advanced Institute of Tec**
**Gyeonggi-do (KR)**

(74) Representative: **Grünecker Patent- und Rechtsanwälte**
**PartG mbB**
**Leopoldstraße 4**
**80802 München (DE)**

(56) References cited:
**EP-A- 1 217 364**

- **"THE HOW AND WHY OF ELECTRONIC NOSES" IEEE SPECTRUM, IEEE INC. NEW YORK, US, vol. 35, no. 9, September 1998 (1998-09), pages 22-34, XP000848941 ISSN: 0018-9235**
- **CRAVEN M A ET AL: "Electronic noses - development and future prospects" TRAC, TRENDS IN ANALYTICAL CHEMISTRY, ELSEVIER, AMSTERDAM, NL, vol. 15, no. 9, October 1996 (1996-10), pages 486-493, XP004708527 ISSN: 0165-9936**
- **GARDNER J W ET AL: "DETECTION OF VAPOURS AND ODOURS FROM A MULTISENSOR ARRAY USING PATTERN-RECOGNITION TECHNIQUES. ÖPART 2. ARTIFICIAL NEURAL NETWORKS" SENSORS AND ACTUATORS B, ELSEVIER SEQUOIA S.A., LAUSANNE, CH, vol. B09, no. 1, 1 July 1992 (1992-07-01), pages 9-15, XP000297731 ISSN: 0925-4005**
- **HORNER G: "SIGNALVERARBEITUNG BEI CHEMOSENSORARRAYS" TECHNISCHES MESSEN TM, R.OLDENBOURG VERLAG. MUNCHEN, DE, vol. 62, no. 4, 1 April 1995 (1995-04-01), pages 166-172, XP000516380 ISSN: 0171-8096**
- **HINES E L ET AL: "Electronic noses: a review of signal processing techniques" IEE PROCEEDINGS: CIRCUITS DEVICES AND SYSTEMS, INSTITUTION OF ELECTRICAL ENGINEERS, STENVENAGE, GB, vol. 146, no. 6, 6 December 1999 (1999-12-06), pages 297-310, XP006013140 ISSN: 1350-2409**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

BACKGROUND OF THE INVENTION

1. Field of the Invention

The present invention relates to a method and apparatus for simultaneously detecting a length and concentration of a nucleic acid.

2. Description of the Related Art

Transistor-based biosensors are a kind of sensor for detecting ionic materials, especially, biomolecules, using electrical signals. Transistor-based biosensors are manufactured through semiconductor manufacturing processes and have the advantage of high speed electrical signal transition so that many studies involving them have been conducted.

U.S. Patent No. 4,238,757 is the original patent relating to the detection of a biological response using a field effect transistor (FET). This patent relates to a biosensor detecting an antigen-antibody reaction by way of a current change of semiconductor inversion layer occurring due to a change in surface charge concentration. This patent relates to the detection of a protein among biomolecules.

The advantages of using a FET as a biosensor are lower costs, high-speed sensing, and a simpler combination of integrated circuit (IC)/MEMS process, compared to conventional methods.

On a surface of a gate electrode of a FET based biosensor, probe biomolecules may be fixed or not. A detecting method for biomolecules using a FET-based biosensor involves a measuring current or voltage change due to the binding of a target biomolecule to the surface of the gate electrode to which probe biomolecules can be fixed or not. In an alternative method, the detecting method can be performed by measuring the current or voltage change due to the presence of a target biomolecule within a certain distance from a gate electrode to which no probe biomolecules are fixed.

However, when conventional FET-based biosensors are used to detect a target ionic material, the biosensors can detect only a concentration of unspecified ionic materials due to a voltage or current change, however, there is a problem in that it cannot be ascertained whether the target ionic materials is responsible for the voltage or current change.

For example, when a primer is accurately selected and assay conditions are accurately set in order that a specific polymerase chain reaction (PCR) should be ideally performed in advance, the existence of target nucleic acid can be fully confirmed from only the existence of bands. However, when PCR is actually performed using an unknown sample, reactants obtained under second-best conditions instead of the optimum conditions are detected, and thus errors can frequently occur.

Therefore, there is a need for simultaneously measuring the concentration and size of ionic particles, for example, a length of nucleic acid in order to determine whether an error has occurred or not.

The document "THE HOW AND WHY OF ELECTRONIC NOSES" IEEE SPECTRUM, IEEE INC. NEW YORK, US, vol. 35, no. 9, September 1998 (1998-09), pages 22-34, discloses a system that uses gas sensors to identify an odor using a MOSFET based on changes in its electrical properties by odorant reaction products that penetrate the gate.

CRAVEN M A ET AL: "Electronic noses - development and future prospects" TRAC, TRENDS IN ANALYTICAL CHEMISTRY, ELSEVIER, AMSTERDAM, NL, vol. 15, no. 9, October 1996 (1996-10), pages 486-493, disclose an array of chemical sensors for detecting odorant molecules based on voltage changes in a MOS chemoresistive sensor.

GARDNER J W ET AL: "DETECTION OF VAPOURS AND ODOURS FROM A MULTISENSOR ARRAY USING PATTERN-RECOGNITION TECHNIQUES. OPART 2. ARTIFICIAL NEURAL NETWORKS" SENSORS AND ACTUATORS B, ELSEVIER SEQUOIA S.A., LAUSANNE, CH, vol. B09, no. 1, 1 July 1992 (1992-07-01), pages 9-15, disclose the use of an array of gas sensors to identify vapors and odors, including parametric expressions of sensor response such as fractional conductance change and relative conductance.

HORNER G: "SIGNALVERARBEITUNG BEI CHEMOSENSORARRAYS" TECHNISCHES MESSEN TM, R.OLDENBOURG VERLAG. MUNCHEN, DE, vol. 62, no. 4, 1 April 1995 (1995-04-01), pages 166-172, discloses integrated sensor arrays, the signals of which can be used to identify and quantify gases and odors with pattern recognition methods.

HINES E L ET AL: "Electronic noses: a review of signal processing techniques" IEE PROCEEDINGS: CIRCUITS DEVICES AND SYSTEMS, INSTITUTION OF ELECTRICAL ENGINEERS, STENVENAGE, GB, vol. 146, no. 6, 6 December 1999 (1999-12-06), pages 297-310, review signal processing techniques, for example steady state techniques for static signal values from FET-based odor sensors.

SUMMARY OF THE INVENTION

The present invention provides a method of detecting a length and concentration of a nucleic acid in a solution according to claim 1.

The present invention also provides an apparatus for detecting a length and concentration of nucleic acids according to claim 9.

Specific embodiments are defined in the dependent claims.

## BRIEF DESCRIPTION OF THE DRAWINGS

The above and other features and advantages of the present invention will become more apparent by describing in detail exemplary embodiments thereof with reference to the attached drawings in which:

FIG. 1 is a flowchart illustrating a method of detecting a length and concentration of a nucleic acid according to an embodiment of the present invention;
FIG. 2 is a graph conceptually illustrating a process of determining a cross point between equipotential lines, according to an embodiment of the present invention;
FIG. 3 is a block diagram schematically illustrating a constitution of an apparatus for detecting a lenght and concentration of a nucleic acid according to an embodiment of the present invention;
FIG. 4 is a side cross-sectional view schematically illustrating FET-based sensors included in a voltage drop measurement unit of FIG. 3 according to an embodiment of the present invention;
FIG. 5 is a side cross-sectional view schematically illustrating FET-based sensors included in a voltage drop measurement unit of FIG. 3 according to another embodiment of the present invention; and
FIG. 6 is a top view schematically illustrating FET-based sensors included in a voltage drop measurement unit of FIG. 3 according to another embodiment of the present invention.

## DETAILED DESCRIPTION OF THE INVENTION

Hereinafter, the present invention will be described in greater detail with reference to accompanying drawings.

FIG. 1 is a flowchart illustrating a method of detecting a length and concentration of a nucleic acid according to an embodiment of the present invention.

Referring to FIG. 1, first, measurement of voltage drop values of at least three kinds of nucleic acids of which lengths and concentrations are known 11 is performed using each of at least two FET-based sensors having different electrical characteristics.

The measurement of voltage drop values of the nucleic acids 11 may be performed by introducing solutions each containing one of the nucleic acids in a chamber including at least two FET-based sensors having different electrical characteristics for detecting nucleic acids.

The different electrical characteristics of the FET-based sensors for detecting nucleic acids may be obtained through a difference of a surface material of the sensor, a sensor structure or a size of the sensor. The difference of the sensor structure may be a slight difference that occurs in a process of manufacturing the same sensor.

The FET-based sensors for detecting nucleic acid can independently consist of a plurality of FET-based sensors having the same electrical characteristics. In this case, voltage drop values of the nucleic acids can be calculated by averaging voltage drop values measured using a plurality of FET-based sensors having the same electrical characteristics.

The FET can be any FET that has been used in a conventional bio sensor or CMOS device, etc., and can be both n-MOS and p-MOS. For example, when the substrate is n-type doped, the source and drain can be independently p-type doped, on the other hand, when the substrate is p-type doped, the source and drain can be independently n-type doped.

The nucleic acids can be selected from the group consisting of DNA, RNA, PNA, LNA and mixtures thereof.

Referring back to FIG. 1, next, determination of at least three points in a three-dimensional plot consisting of a length, a concentration and a voltage drop value from the known lengths and concentrations and the measured voltage drop values corresponding to at least the three kinds of nucleic acids 12 is performed with respect to each of at least two FET-based sensors having different electrical characteristics for detecting the nucleic acids.

The three-dimensional plot can comprise, for example, an x-axis set to length, a y-axis set to concentration, and a z-axis set to voltage drop value. The determination of at least the three points 12 can be performed such that first, a voltage drop value is determined using a kind of a nucleic acid of which a length and concentration are known, and the length, concentration and voltage drop value are represented in the three-dimensional plot, and next, points of the remaining two kinds of nucleic acids are represented in the three-dimensional plot as the same method described above.

Then, approximation of a single plane using least the three points 13 is performed with respect to at least two FET-based sensors having different electrical characteristics for detecting nucleic acids.

The plane approximation operation 13 may include any statistical method of plane approximation that is known to those skilled in the art, and is not particularly limited.

For example, when three points are approximated into a single plane, the plane approximation can be performed by solving three constants, such as a, b and c of Equation 1. By this, Equation 1 comprising three variables, such as x, y and z can be obtained.

Equation (1)

$$ax + by + cz + 1 = 0$$

where x refers to the length of nucleic acids, y refers to concentration of the nucleic acids and z refers to voltage drop value of the nucleic acids.

Then, measurement of voltage drop value of a nucleic acid of which length and concentration are unknown 14 is performed using each of at least two FET-based sensors having different electrical characteristics for detecting nucleic acids.

The measurement of voltage drop value of the nucleic acid 14 can be performed by introducing solutions each containing one of the nucleic acids in a chamber including at least two FET-based sensors having different electrical characteristics for detecting nucleic acids.

The FET-based sensors for detecting nucleic acids can independently consist of a plurality of FET-based sensors having the same electrical characteristics. In this case, calculation of voltage drop values of the nucleic acids can be performed by averaging voltage drop values calculated using a plurality of FET-based sensors having the same electrical characteristics.

Next, determination of equipotential lines that exist on the approximated plane 15 is performed using voltage drop values of the unknown nucleic acids with respect to each of at least two FET-based sensors having different electrical characteristics for detecting nucleic acids.

For example, when three points are approximated using a plane, determining the equipotential lines 15 can be performed by substituting z, a voltage drop value of the unknown calculated nucleic acids, into Equation 1. By this, an equation comprising two variables of x and y can be obtained.

Then, determination of a cross point between at least the two equipotential lines 16 is performed.

FIG. 2 is a graph conceptually illustrating a process of determining a cross point between equipotential lines, according to an embodiment of the present invention Referring to FIG. 2, equipotential lines are represented on a two-dimensional plane consisting of DNA length (bp) on the x-axis and concentration on the y-axis. In the case of FET A, 15, 20, 25, 30 and 35 mV equipotential lines are illustrated, and in the case of FET B, 12, 14, 16, 18, 20 and 22 mV equipotential lines are illustrated. When voltage drop values of DNA of which length and concentration are unknown with respect to FET A and FET B having different electrical characteristics for detecting nucleic acids are 30 mV and 18 mV, two equipotential lines which cross are obtained and a cross point thereof is determined using the results. An x value and a y value of the cross point refers to the unknown DNA length (bp) and the unknown DNA concentration, respectively.

Determination of the cross point can be performed such that when at least three equipotential lines exist, cross points between each two equipotential lines of the three equipotential lines are obtained, and an average of the cross points is obtained.

FIG. 3 is a block diagram schematically illustrating a constitution of an apparatus for detecting a length and concentration of a nucleic acid according to an embodiment of the present invention.

Referring to FIG. 3, an apparatus according to an embodiment of the present invention includes a voltage drop measurement unit 31, a point determination unit 32, a plane approximation unit 33, an equipotential line determination unit 34 and a cross point determination unit 35.

The voltage drop measurement unit 31 includes at least two FET-based sensors having different electrical characteristics for detecting nucleic acids and measures voltage drop values of the nucleic acids.

In addition, the voltage drop measurement unit 31 can include at least the two FET-based sensors having different electrical characteristics for detecting nucleic acids in a single chamber.

The different electrical characteristics of at least the two FET-based sensors for detecting nucleic acids may be obtained through a difference of surface materials of the sensor, a sensor structure or a size of the sensor. The difference of the sensor structure may be a slight difference that occurs in a process of manufacturing the same sensor.

In addition, each FET-based sensor for detecting nucleic acid can consist of a plurality of FET-based sensors having the same electrical characteristics.

The FET can be any FET that has been used in a conventional bio sensor or CMOS device, etc., and can be both n-MOS and p-MOS. For example, when the substrate is n-type doped, the source and drain can be independently p-type doped, on the other hand, when the substrate is p-type doped, the source and drain can be independently n-type doped.

The FET can include a substrate; a source and a drain that are formed on either side of the substrate and doped to have the opposite polarity to the substrate; and a gate formed on the substrate to contact the source and the drain. The source supplies carriers which for example may be free electrons or holes, the drain is a site which the supplied carriers from the source reach, and the gate plays a role of controlling the flow of the carriers between the source and the drain. When detecting a nucleic acid within an electrolyte, the FET is the most preferred form of a sensor and it is able to detect the existence of the nucleic acid without labeling (Label-free).

A chamber including the FET can further comprise an inlet and an outlet for the solution, and a micropump for allowing the solution to flow into and out of the chamber.

The nucleic acid can be selected from the group consisting of DNA, RNA, PNA, LNA and mixtures thereof.

FIG. 4 is a side cross-sectional view schematically illustrating FET-based sensors included in the voltage drop measurement unit of FIG. 3 according to an embodiment of the present invention. Referring to FIG. 4, two FET-based sensors 42 and 43 having different electrical characteristics for detecting nucleic acids are included in a single chamber 41. A sample solution containing nucleic acids can flow in the direction of the arrow.

FIG. 5 is a side cross-sectional view schematically illustrating FET-based sensors included in the voltage drop measurement unit of FIG. 3 according to another embodiment of the present invention. Referring to FIG. 5, three FET-based sensors 52, 53 and 54 having different electrical characteristics for detecting nucleic acids is included in a single chamber 51.

FIG. 6 is a top view schematically illustrating FET-based sensors included in the voltage drop measurement unit of FIG. 3 according to another embodiment of the present invention. Referring to FIG. 6, a plurality of FET-based sensors comprise an A group of FET-based sensors (A1,A2,A3,..,Am), a B group of FET-based sensors (B1,B2,B3,..,Bm), a C group of FET-based sensors (C1,C2,C3,..,Cm), etc. All the FET-based sensors of a single group have the same electrical characteristics, however different groups of the FET-based sensors have different electrical characteristics.

The point determination unit 32 determines points in a three-dimensional plot consisting of a lenght, a concentration and a voltage drop value from known lengths, concentrations, and voltage drop values of nucleic acids measured in the voltage drop measurement unit.

In the plane approximation unit 33, at least three points determined by the point determination unit are approximated with a single plane.

The plane approximation unit 33 can perform a process of solving constants of a, b and c of Equation 1 using the three points.

Equation (1)

$$ax + by + cz + 1 = 0$$

where x refers to length of nucleic acids, y refers to concentration and z refers to voltage drop value.

The equipotential line determination unit 34 determines equipotential lines existing on a plane formed in the plane approximation unit using voltage drop value measured in the voltage drop measurement unit of ionic materials of which length and concentration are unknown.

The equipotential line determination unit 34 can perform a process of substituting voltage drop values of the unknown measured nucleic acids into Equation 1.

The cross point determination unit 35 determines a cross point between at least two equipotential lines determined by the equipotential line determination unit.

The cross point determination unit 35 can perform a process such that when at least three equipotential lines exist, cross points between each two equipotential lines of the three equipotential lines are obtained, and an average point of the cross points is obtained.

Hereinafter, the present invention will be described in further detail with reference to the following examples. These examples are for illustrative purposes only and are not intended to limit the scope of the present invention.

Example 1

Manufacture of a FET-based biosensor

The FET element used in the current example was customized from X-FAB Semiconductor Foundries (Germany). The customized product is an array in which 48 (4 x 12) FL FETs manufactured to all have the same structure and little electrical characteristic differences are disposed, and an array in which 192 (12 x 16) NMOS FETs manufactured to all have the same structure and little electrical characteristic differences are disposed, wherein, however, the structures and electrical characteristics of the NMOS FETs differ from those of the FL FETs. The product was manufactured by using the facility of X-FAB Semiconductor Foundries corp., and by performing the intrinsic CMOS process of the company. The standard CMOS process of each company has slight differences, however, is the details thereof are omitted since it does not have a big effect on the characteristics of the FET element and has no concern with the present invention.

By removing a passivation layer and a gate electrode layer of the each FET in the arrays, a polysilicon layer was exposed. Then, the FET surface including the exposed polysilicon layer was carefully washed. The washing was performed with pure acetone and water, and after washing, the resulting products were dried. The substrate washing process used the wet station which is used in a semiconductor manufacturing process. After the washing was over, the product was dried using a spin dry.

A chamber side wall was installed on the array substrate and the substrate was coated with platinum as a standard electrode. A chamber A including 12 FL FETs and 36 NMOS FETs, a chamber B including 12 FL FETs, and a chamber C including 36 NMOS FETs were independently manufactured.

Experimental Example 1

Detecting a length and concentration of DNA using the method according to an embodiment of the present invention

By using each chamber A, B and C manufactured in Example 1, confirmation of whether a length and concentration of a target DNA can be accurately measured was obtained.

DNA having a concentration of 12 ng/$\mu\ell$ and a length of 249 bp was used as the target DNA.

1-1: Using two different FET groups

A method according to FIG. 1 was performed using the 12 FL FETs of chamber A as one group and the 36 NMOS FETs of chamber A as another group.

That is, voltage drop values of three kinds of DNAs of which length and concentration were known with respect to each group were measured. Then, voltage drop values of each DNA measured in 12 FL FETs were averaged, and voltage drop values of each DNA measured in 36 NMOS FETs were averaged.

Next, 3 points were determined in a three-dimensional plot comprising a length (x), a concentration (y) and voltage drop (z) with respect to the group of FL FETs, and a single plane was approximated from them. In particular, when known lengths and concentrations of the three kinds of DNAs were independently x1, y1; x2, y2; and x3, y3, and measure voltage drop values were z1, z2 and z3, respectively, a plane equation of a1x + b1y + c1z + 1 = 0 was obtained by simultaneously solving three equations, that is a1x1 + b1y1 + c1z1 + 1 = 0; a1x2 + b1y2 + c1z2 + 1 = 0; and a1x3 + b1y3 + c1z3 + 1 = 0 and thus determining the values of a, b and c.

Likewise, another plane equation of a2x + b2y + c2z + 1 = 0 was obtained by performing the same method with respect to the group of NMOS FETs.

Thereafter, voltage drop values of target DNAs of which length and concentration were to be detected with respect to each group were measured and averaged to obtain z4 and z5, and those values were substituted into the plane equation. Accordingly, two equations of equipotential lines, that is a1x + b1y + c1z4 + 1 = 0 and a2x + b2y + c2z5 + 1 = 0 were obtained, the two equations were simultaneously solved, and thus x and y values of one cross point were obtained to obtain a length and concentration of the target DNAs.

The results are shown in Table 1.

1-2: Using 12 FETs

A method was performed in the same manner as in Experimental Example 1-1 by individually using each different FET, supposing that each 12 FL FET included the chamber B had little electrical characteristic differences from the manufacturing process.

As a result, 12 equipotential lines and 132 (12 x 11) cross points were obtained. Accordingly x and y values of the cross points were averaged to obtain a length and concentration of target DNAs. The results are shown in Table 1.

1-3: Using 36 FETs

A method was performed in the same manner as in Experimental Example 1-1 by individually using each different FET, supposing that each 36 NMOS FETs included in the chamber C had little electrical characteristic differences from the manufacturing process.

As a result, 36 equipotential lines and 1,260 (36 x 35) cross points were obtained. Accordingly x and y values of the cross points were averaged to obtain a length and concentration of target DNAs. The results are shown in Table 1.

1-4: Using 48 FETs

A method was performed in the same manner as in Experimental Example 1-1 by individually using each different FET, supposing that each 12 FL FET and 36 NMOS FET included in the chamber A had little electrical characteristic differences from a manufacturing process.

As a result, 48 equipotential lines and 2,256 (48 x 47) cross points were obtained. Accordingly x and y values of the cross points were averaged to obtain a length and concentration of target DNAs. The results for examples 1-1 to 1-4 according to the invention are shown in Table 1.

Table 1

| Experimental Example | x: length (bp) | y: concentration (ng/$\mu\ell$) |
|---|---|---|
| 1-1 | 321.067621 | 36.743186 |
| 1-2 | 756.196412 | -13.164146 |
| 1-3 | 124.486370 | 12.876566 |
| 1-4 | 264.433197 | 7.496748 |
| Real length | 249 | 12 |

Best results were obtained with example 1-4 using two types of FETs having different electrical properties and individually using each FET.

The present invention can be embodied as computer readable codes on a computer readable recording medium. The computer readable recording medium is any data storage device that can store data which can be thereafter read by a computer system. Examples of the computer readable recording medium include read-only memory (ROM), random-access memory (RAM), CD-ROMs, magnetic tapes, floppy disks, optical data storage devices, and carrier waves (such as data transmission through the Internet).

As described above, a concentration and length of nucleic acids can be simultaneously measured according to the present invention.

While the present invention has been particularly shown and described with reference to exemplary embodiments thereof, it will be understood by those of ordinary skill in the art that various changes in form and details may be made therein without departing from the scope of the present invention as defined by the following claims.

## Claims

**1.** A method of simultaneously detecting a length and concentration of a nucleic acid in a solution, the method comprising:

measuring voltage drop values of at least three kinds of nucleic acids of which the lengths and concentrations are known with respect to each of at least two FET-based sensors having different electrical characteristics for detecting nucleic acids;
determining at least three points in a three-dimensional plot consisting of a length, a concentration and a voltage drop value from the known lengths, concentrations, and the measured voltage drop values of at least three kinds of nucleic acids with respect to each of at least the two FET-based sensors;
approximating the determined at least three points to a single plane with respect to each of the at least two FET-based sensors;
measuring a voltage drop value of a nucleic acid of which length and concentration are unknown with respect to each of the at least two FET-based sensors;
determining equipotential lines existing on the plane using the voltage drop value of the unknown nucleic acid with respect to each of the at least two FET-based sensors;
determining at least one cross point between the determined equipotential lines; and
determining simultaneously the unknown length and concentration of the nucleic acid from the at least one cross point.

**2.** The method of claim 1, wherein the measuring of voltage drop values of the nucleic acids is performed by introducing a solution containing each nucleic acid into a chamber (41) including at least two FET-based sensors (42, 43) having different electrical characteristics for detecting the nucleic acids.

**3.** The method of claim 1 or 2, wherein the different electrical characteristics of the at least two FET-based sensors for detecting nucleic acids are obtained through a difference of materials on a surface of the sensor, a sensor structure or a size of the sensor.

**4.** The method according to one of claims 1 to 3, wherein each of the at least two FET-based sensors for detecting nucleic acids comprises a plurality of FET-based sensors (A1, A2,...,Am) having the same electrical characteristics.

**5.** The method of claim 4, wherein the measuring of the voltage drop values of the nucleic acids is performed by averaging voltage drop values measured using a plurality of FET-based sensors (A1, A2,...Am) having the same electrical characteristics.

**6.** The method according to one of claims 1 to 5, wherein the approximating of the plane is performed by solving constants of a, b and c of Equation 1 using three points:

Equation (1)

$$ax + by + cz + 1 = 0$$

where x refers to length of nucleic acids, y refers to concentration and z refers to voltage drop value.

**7.** The method of claim 6, wherein the determining of the equipotential lines is performed by substituting voltage drop value of the unknown measured nucleic acid into Equation 1.

**8.** The method according to one of claims 1 to 7, wherein the determining of a cross point between the equipotential lines is performed such that when at least three equipotential lines exist, cross points between each two equipotential lines of the equipotential lines are obtained, and an average point of the cross points is obtained.

**9.** An apparatus for simultaneously detecting a length and concentration of nucleic acids, the apparatus comprising:

a voltage drop measurement unit (31) that includes at least two FET-based sensors having different electrical characteristics for detecting nucleic acids;
a point determination unit (32) that is adapted to determine at least three points in a three-dimensional plot consisting of a length, a concentration and a voltage drop value from the known length, concentration, and voltage drop value measured in the voltage drop measurement unit (31) of at least three kinds of nucleic acids using each of the at least two FET-based sensors;
a plane approximation unit (33) that is adapted to approximate the at least three points determined in the point determination unit (32) into a single plane;
an equipotential line determination unit (34) that is adapted to determine equipotential lines existing on a plane approximated in the plane approximation unit (33) using voltage drop values measured in the voltage drop measurement unit (31) of nucleic acids of which length and concentration are unknown; and
a cross point determination unit (35) that is adapted to determine a cross point between at least two equipotential lines determined in the equipotential line determination unit (34), wherein the cross point is to be used for simultaneously determining the unknown length and concentration of the nucleic acid.

**10.** The apparatus of claim 9, wherein the voltage drop measurement unit (31) comprises at least two FET-based sensors (42, 43) having different electrical characteristics for detecting nucleic acids in a single chamber (41).

**11.** The apparatus of claim 9 or 10, wherein the different electrical characteristics of the at least two FET-based sensors (42, 43) for detecting nucleic acids are obtained through a difference of materials on a surface of the sensor, a sensor structure or a size of the sensor.

**12.** The apparatus according to one of claims 9 to 11, wherein each of at least two FET-based sensors for detecting nucleic acids comprises a plurality of FET-based sensors (A1, A2,...,Am) having the same electrical characteristics.

**13.** The apparatus according to one of claims 9 to 12, wherein the plane approximation unit (33) is configured to perform a process of solving constants of a, b and c of Equation 1 using three points:

Equation (1)

$$ax + by + cz + 1 = 0$$

where x refers to length of nucleic acids, y refers to concentration and z refers to voltage drop value.

**14.** The apparatus of claim 13, wherein the equipotential line determination unit (34) is configured to perform a process of substituting voltage drop value of the unknown measured nucleic acids into Equation 1.

**15.** The apparatus according to one of claims 9 to 14, wherein the cross point determination unit (35) is configured to perform a process such that when at least three equipotential lines exist, cross points between each two equipotential lines of the equipotential lines are obtained, and an average point of the cross points is obtained.

**Patentansprüche**

**1.** Verfahren zum gleichzeitigen Erfassen einer Länge und Konzentration einer Nukleinsäure in einer Lösung, wobei das Verfahren umfasst:

Messen von Spannungsabfallwerten von wenigstens drei Arten von Nukleinsäuren, deren Längen und Konzentrationen in Bezug auf jeden von wenigstens zwei Sensoren auf FET-Basis mit unterschiedlichen elektrischen Eigenschaften zum Erfassen von Nukleinsäuren bekannt sind;
Bestimmen von wenigstens drei Punkten in einem dreidimensionalen Diagramm, bestehend aus einer Länge, einer Konzentration und einem Spannungsabfallwert aus den bekannten Längen, Konzentrationen und den gemessenen Spannungsabfallwerten von wenigstens drei Arten von Nukleinsäuren in Bezug auf jeden der wenigstens zwei FET-basierten Sensoren;
Annähern der bestimmten wenigstens drei Punkte an eine einzige Ebene in Bezug auf jeden der wenigstens zwei FET-basierten Sensoren;
Messen eines Spannungsabfallwertes einer Nukleinsäure, deren Länge und Konzentration in Bezug auf jeden der wenigstens zwei Sensoren auf FET-Basis unbekannt sind;
Bestimmen von in der Ebene vorhandenen Äquipotentiallinien unter Verwendung des Spannungsabfallwertes der unbekannten Nukleinsäure in Bezug auf jeden der wenigstens zwei Sensoren auf FET-Basis;
Bestimmen von wenigstens einem Kreuzungspunkt zwischen den bestimmten Äquipotentiallinien; und
gleichzeitiges Bestimmen der unbekannten Länge und Konzentration der Nukleinsäure von dem wenigstens einen Kreuzungspunkt.

**2.** Verfahren nach Anspruch 1, bei dem das Messen der Spannungsabfallwerte der Nukleinsäuren durch Einführen einer Lösung, die jede Nukleinsäure enthält, in eine Kammer (41) umfasst, die wenigstens zwei FET-basierte Sensoren (42, 43) mit unterschiedlichen elektrischen Eigenschaften zum Nachweis der Nukleinsäuren hat.

**3.** Verfahren nach Anspruch 1 oder 2, bei dem man die unterschiedlichen elektrischen Eigenschaften der wenigstens zwei FET-basierten Sensoren zum Erfassen von Nukleinsäuren durch einen Unterschied der Materialien auf einer Oberfläche des Sensors, einer Sensorstruktur oder einer Größe des Sensors erhält.

**4.** Verfahren nach einem der Ansprüche 1 bis 3, bei dem jeder der wenigstens zwei FET-basierten Sensoren zum Erfassen von Nukleinsäuren eine Vielzahl von FET-basierten Sensoren (A 1, A2, ..., Am) umfasst, die dieselben elektrischen Eigenschaften haben.

**5.** Verfahren nach Anspruch 4, bei dem das Messen der Spannungsabfallwerte der Nukleinsäuren durch Mitteln von Spannungsabfallwerten unter Verwendung einer Vielzahl von Sensoren auf FET-Basis (A 1, A2, ... Am) ausgeführt wird, die dieselben elektrischen Eigenschaften haben.

**6.** Verfahren nach einem der Ansprüche 1 bis 5, bei dem das Annähern der Ebene durch Lösen von Konstanten von a, b und c von Gleichung 1 unter Verwendung von drei Punkten ausgeführt wird:

Gleichung (1)

$$ax + by + cz + 1 = 0,$$

wobei sich x auf die Länge der Nukleinsäuren bezieht, y sich auf die Konzentration bezieht und z sich auf den Spannungsabfallwert bezieht.

**7.** Verfahren nach Anspruch 6, bei dem das Bestimmen der Äquipotentiallinien durch Ersetzen des Spannungsabfallwertes der unbekannten gemessenen Nukleinsäure in die Gleichung 1 ausgeführt wird.

**8.** Verfahren nach einem der Ansprüche 1 bis 7, bei dem das Bestimmen eines Kreuzungspunktes zwischen den Äquipotentiallinien derart ausgeführt wird, dass man, wenn wenigstens drei Äquipotentiallinien vorhanden sind, Kreuzungspunkte zwischen jeweils zwei Äquipotentiallinien der Äquipotentiallinien erhält und man einen Durchschnittspunkt der Kreuzungspunkte erhält.

**9.** Vorrichtung zum gleichzeitigen Erfassen einer Länge und Konzentration von Nukleinsäuren, wobei die Vorrichtung umfasst:

eine Spannungsabfallmesseinheit (31), die wenigstens zwei FET-basierte Sensoren mit unterschiedlichen elektrischen Eigenschaften zum Nachweis von Nukleinsäuren umfasst;
eine Punktbestimmungseinheit (32), die dazu eingerichtet ist, wenigstens drei Punkte in einem dreidimensionalen Diagramm zu bestimmen, das aus einer Länge, einer Konzentration und einem Spannungsabfallwert aus dem bekannten Längen-, Konzentrations- und Spannungsabfallwert besteht, der in der Spannungsabfall-Messeinheit (31) von wenigstens drei Arten von Nukleinsäuren unter Verwendung jedes der wenigstens zwei Sensoren auf FET-Basis gemessen wird;
eine Ebenenannäherungseinheit (33), die dazu eingerichtet ist, die wenigstens drei Punkte, die in der Punktbestimmungseinheit (32) bestimmt werden, in eine einzige Ebene anzunähern;
eine Äquipotentiallinien-Bestimmungseinheit (34), die dazu eingerichtet ist, Äquipotentiallinien, die in einer in der Näherungseinheit (33) angenäherten Ebene vorliegen, unter Verwendung von Spannungsabfallwerten zu bestimmen, die in der Spannungsabfallmesseinheit (31) von Nukleinsäuren gemessen werden, deren Länge und Konzentration unbekannt sind; und
eine Kreuzungspunktbestimmungseinheit (35), die dazu eingerichtet ist, einen Kreuzungspunkt zwischen wenigstens zwei Äquipotentiallinien zu bestimmen, die in der Äquipotentiallinien-Bestimmungseinheit (34) bestimmt werden, wobei der Kreuzungspunkt zur gleichzeitigen Bestimmung der unbekannten Länge und Konzentration der Nukleinsäure verwendet werden soll.

**10.** Vorrichtung nach Anspruch 9, bei der die Spannungsabfallmesseinheit (31) wenigstens zwei FET-basierte Sensoren (42, 43) mit unterschiedlichen elektrischen Eigenschaften zum Nachweis von Nukleinsäuren in einer einzelnen Kammer (41) umfasst.

**11.** Vorrichtung nach Anspruch 9 oder 10, bei der man die unterschiedlichen elektrischen Eigenschaften der wenigstens zwei FET-basierten Sensoren (42, 43) zum Erfassen von Nukleinsäuren durch einen Unterschied von Materialien auf einer Oberfläche des Sensors, einer Sensorstruktur, oder einer Größe des Sensors erhält.

**12.** Vorrichtung nach einem der Ansprüche 9 bis 11, bei der jeder von wenigstens zwei FET-basierten Sensoren zum Erfassen von Nukleinsäuren eine Vielzahl von FET-basierten Sensoren (A 1, A2, ..., Am) umfasst, die dieselben elektrischen Eigenschaften aufweisen.

**13.** Vorrichtung nach einem der Ansprüche 9 bis 12, bei der die Ebenenannäherungseinheit (33) dazu eingerichtet ist, einen Vorgang zum Lösen von Konstanten von a, b und c von Gleichung 1 unter Verwendung von drei Punkten durchzuführen:

Gleichung (1)

$$ax + by + cz + 1 = 0,$$

wobei sich x auf die Länge der Nukleinsäuren bezieht, y sich auf die Konzentration bezieht und z sich auf den Spannungsabfallwert bezieht.

**14.** Vorrichtung nach Anspruch 13, bei der die Äquipotentiallinien-Bestimmungseinheit (34) dazu eingerichtet ist, einen Vorgang des Ersetzens eines Spannungsabfallwerts der unbekannten gemessenen Nukleinsäuren in die Gleichung 1 auszuführen.

**15.** Vorrichtung nach einem der Ansprüche 9 bis 14, bei der die Kreuzungspunktbestimmungseinheit (35) dazu eingerichtet ist, einen Vorgang derart auszuführen, dass man, wenn wenigstens drei Äquipotentiallinien vorhanden sind, Kreuzungspunkte zwischen jeweils zwei Äquipotentiallinien der Äquipotentiallinien erhält, und man einen Durchschnittspunkt der Kreuzungspunkte erhält.

**Revendications**

**1.** Procédé de détection simultanée de la longueur et de la concentration d'un acide nucléique dans une solution, le procédé consistant à :

mesurer des valeurs de chute de potentiel d'au moins trois sortes d'acides nucléiques dont les longueurs et les concentrations sont connues par rapport à chacun d'au moins deux capteurs à base de transistor à effet de champ ayant des caractéristiques électriques différentes pour la détection d'acides nucléiques ;
déterminer au moins trois points dans un graphe tridimensionnel consistant en une longueur, une concentration et une valeur de chute de potentiel à partir des longueurs et des concentrations connues ainsi que des valeurs de chute de potentiel mesurées d'au moins trois sortes d'acides nucléiques par rapport à chacun des au moins deux capteurs à base de transistor à effet de champ ;
définir par approximation un plan unique passant par les au moins trois points déterminés par rapport à chacun des au moins deux capteurs à base de transistor à effet de champ ;
mesurer une valeur de chute de potentiel d'un acide nucléique dont la longueur et la concentration sont inconnues par rapport à chacun des au moins deux capteurs à base de transistor à effet de champ ;
déterminer des lignes équipotentielles existant sur le plan en utilisant la valeur de chute de potentiel de l'acide nucléique inconnu par rapport à chacun des au moins deux capteurs à base de transistor à effet de champ ;
déterminer au moins un point de croisement entre les lignes équipotentielles déterminées ; et
déterminer simultanément la longueur et la concentration inconnues de l'acide nucléique à partir de l'au moins un point de croisement.

**2.** Procédé selon la revendication 1, dans lequel la mesure des valeurs de chute de potentiel des acides nucléiques est réalisée en introduisant une solution contenant chaque acide nucléique dans une chambre (41) incluant au moins deux capteurs à base de transistor à effet de champ (42, 43) ayant des caractéristiques électriques différentes pour la détection des acides nucléiques.

**3.** Procédé selon la revendication 1 ou 2, dans lequel les caractéristiques électriques différentes des au moins deux capteurs à base de transistor à effet de champ pour la détection d'acides nucléiques sont obtenues grâce à une différence de matériaux sur une surface du capteur, une structure du capteur ou une dimension du capteur.

**4.** Procédé selon l'une des revendications 1 à 3, dans lequel chacun des au moins deux capteurs à base de transistor à effet de champ pour la détection d'acides nucléiques comprend une pluralité de capteurs à base de transistor à effet de champ (A1, A2, ..., Am) ayant les mêmes caractéristiques électriques.

**5.** Procédé selon la revendication 4, dans lequel la mesure des valeurs de chute de potentiel des acides nucléiques est réalisée en formant une moyenne de valeurs de chute de potentiel mesurées en utilisant une pluralité de capteurs à base de transistor à effet de champ (A1, A2, ..., Am) ayant les mêmes caractéristiques électriques.

**6.** Procédé selon l'une des revendications 1 à 5, dans lequel l'approximation du plan s'effectue par résolution des constantes a, b et c de l'Équation 1 en utilisant trois points :

Équation (1)

$$ax + by + cz + 1 = 0$$

où x représente la longueur des acides nucléiques, y représente la concentration et z représente la valeur de la chute de potentiel.

**7.** Procédé selon la revendication 6, dans lequel la détermination des lignes équipotentielles est réalisée en remplaçant la variable par la valeur de chute de potentiel de l'acide nucléique inconnu mesuré dans l'Équation 1.

**8.** Procédé selon l'une des revendications 1 à 7, dans lequel la détermination d'un point de croisement entre les lignes équipotentielles est réalisée en sorte que, lorsqu'il existe au moins trois lignes équipotentielles, des points de croisement entre deux lignes équipotentielles respectives des lignes équipotentielles sont obtenus et un point résultant de la moyenne des points de croisement est obtenu.

**9.** Appareil permettant de détecter simultanément une longueur et une concentration d'acides nucléiques, l'appareil comprenant :

un module de mesure de chute de potentiel (31) qui comprend au moins deux capteurs à base de transistor à effet de champ ayant des caractéristiques électriques différentes pour la détection d'acides nucléiques ;
un module de détermination de points (32) qui est conçu pour déterminer au moins trois points dans un graphe tridimensionnel consistant en une longueur, une concentration et une valeur de chute de potentiel à partir de la longueur et de la concentration connues ainsi que de la valeur de chute de potentiel, mesurée dans le module de mesure de chute de potentiel (31), d'au moins trois sortes d'acides nucléiques en utilisant chacun des au moins deux capteurs à base de transistor à effet de champ ;
un module d'approximation de plan (33) qui est conçu pour définir par approximation un plan unique passant par les au moins trois points déterminés dans le module de détermination de points (32) ;
un module de détermination de lignes équipotentielles (34) qui est conçu pour déterminer des lignes équipotentielles existant sur un plan défini par approximation dans le module d'approximation de plan (33) en utilisant des valeurs de chute de potentiel, mesurées dans le module de mesure de chute de potentiel (31), d'acides nucléiques dont la longueur et la concentration sont inconnues ; et
un module de détermination de point de croisement (35) qui est conçu pour déterminer un point de croisement entre au moins deux lignes équipotentielles déterminées dans le module de détermination de lignes équipotentielles (34), dans lequel le point de croisement est destiné à être utilisé pour la détermination simultanée de la longueur et de la concentration inconnues de l'acide nucléique.

**10.** Appareil selon la revendication 9, dans lequel le module de mesure de chute de potentiel (31) comprend au moins deux capteurs à base de transistor à effet de champ (42, 43) ayant des caractéristiques électriques différentes pour la détection d'acides nucléiques dans une chambre unique (41).

**11.** Appareil selon la revendication 9 ou 10, dans lequel les caractéristiques électriques différentes des au moins deux capteurs à base de transistor à effet de champ (42, 43) pour la détection d'acides nucléiques sont obtenues grâce à une différence de matériaux sur une surface du capteur, une structure du capteur ou une dimension du capteur.

**12.** Appareil selon l'une des revendications 9 à 11, dans lequel chacun des au moins deux capteurs à base de transistor à effet de champ pour la détection d'acides nucléiques comprend une pluralité de capteurs à base de transistor à effet de champ (A1, A2, ..., Am) ayant les mêmes caractéristiques électriques.

**13.** Appareil selon l'une des revendications 9 à 12, dans lequel le module d'approximation de plan (33) est conçu pour réaliser un processus de résolution des constantes a, b et c de l'Équation 1 en utilisant trois points :

Équation (1)

$$ax + by + cz + 1 = 0$$

où x représente la longueur des acides nucléiques, y représente la concentration et z représente la valeur de la chute de potentiel.

**14.** Appareil selon la revendication 13, dans lequel le module de détermination de lignes équipotentielles (34) est conçu pour réaliser un processus de remplacement de la variable par la valeur de chute de potentiel des acides nucléiques inconnus mesurés dans l'Équation 1.

**15.** Appareil selon l'une des revendications 9 à 14, dans lequel le module de détermination de point de croisement (35) est conçu pour réaliser un processus en sorte que, lorsqu'il existe au moins trois lignes équipotentielles, des points de croisement entre deux lignes équipotentielles respectives des lignes équipotentielles sont obtenus et un point résultant de la moyenne des points de croisement est obtenu.

FIG. 1

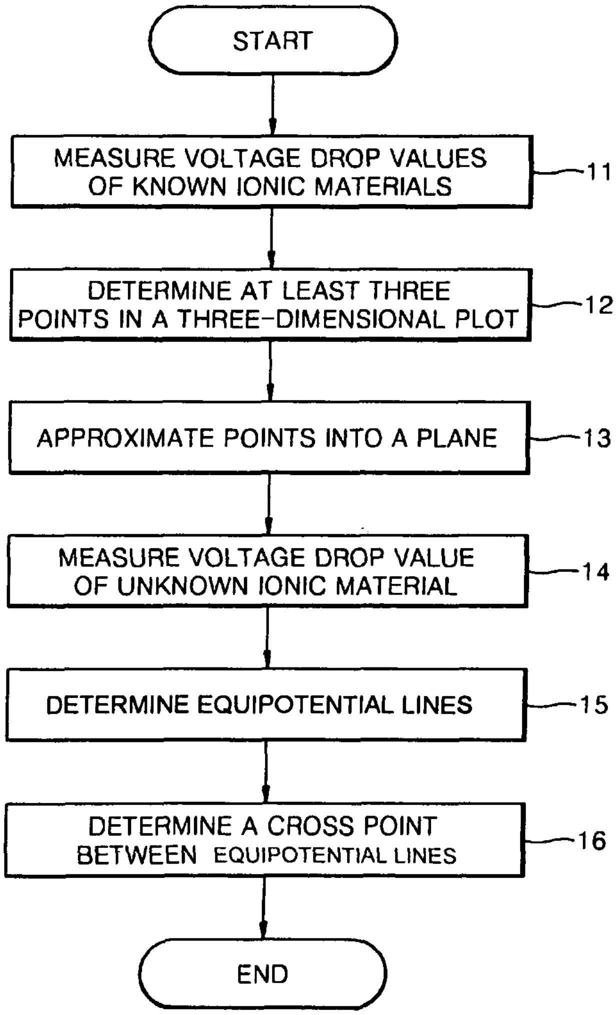
START
MEASURE VOLTAGE DROP VALUES OF KNOWN IONIC MATERIALS
11
DETERMINE AT LEAST THREE POINTS IN A THREE-DIMENSIONAL PLOT
12
APPROXIMATE POINTS INTO A PLANE
13
MEASURE VOLTAGE DROP VALUE OF UNKNOWN IONIC MATERIAL
14
DETERMINE EQUIPOTENTIAL LINES
15
DETERMINE A CROSS POINT BETWEEN EQUIPOTENTIAL LINES
16
END

FIG. 2

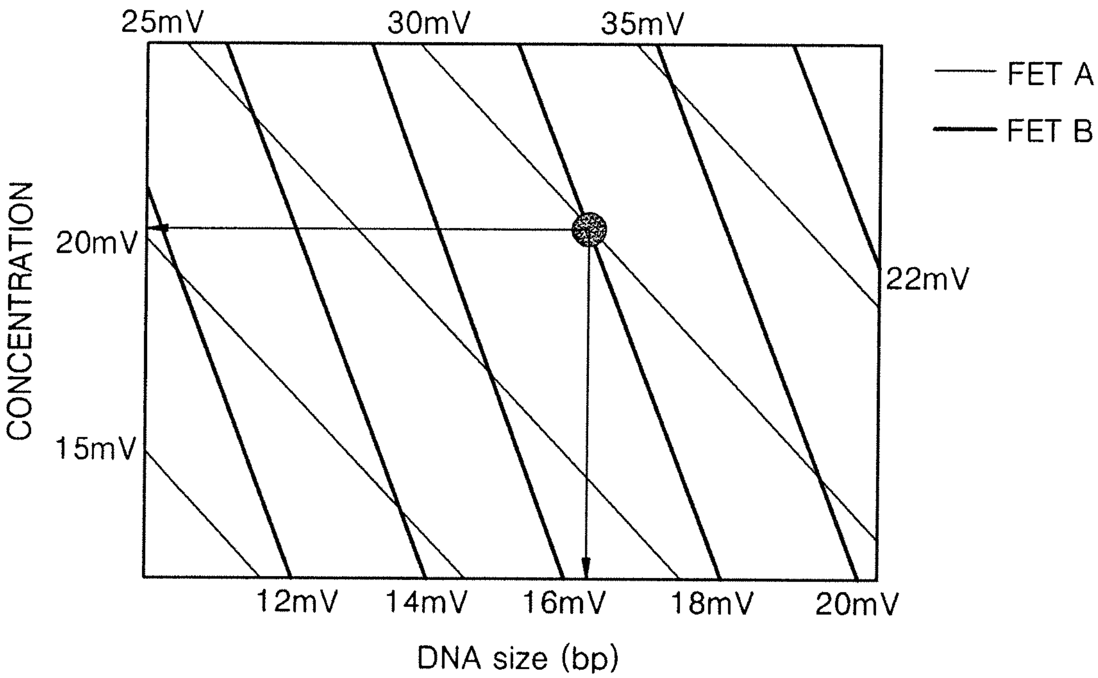
25mV
30mV
35mV
FET A
FET B
CONCENTRATION
20mV
15mV
22mV
12mV
14mV
16mV
18mV
20mV
DNA size (bp)

FIG. 3

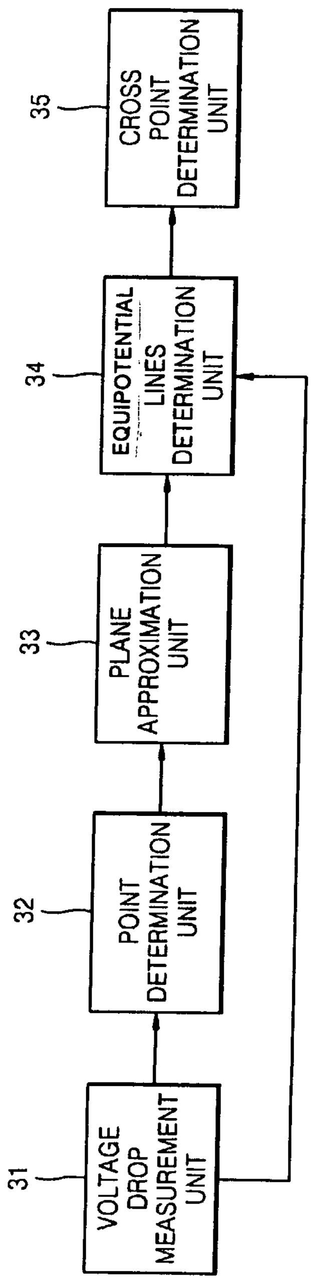
31
VOLTAGE DROP MEASUREMENT UNIT
32
POINT DETERMINATION UNIT
33
PLANE APPROXIMATION UNIT
34
EQUIPOTENTIAL LINES DETERMINATION UNIT
35
CROSS POINT DETERMINATION UNIT

FIG. 4

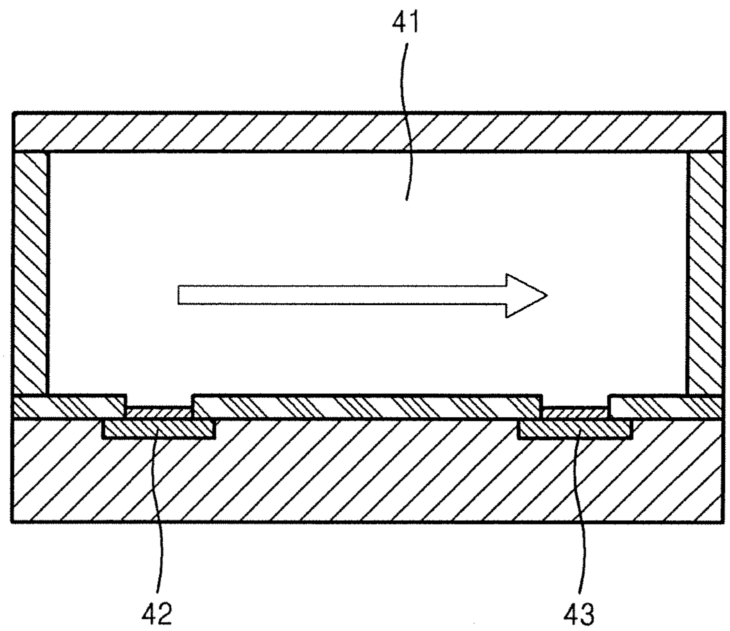


FIG. 5

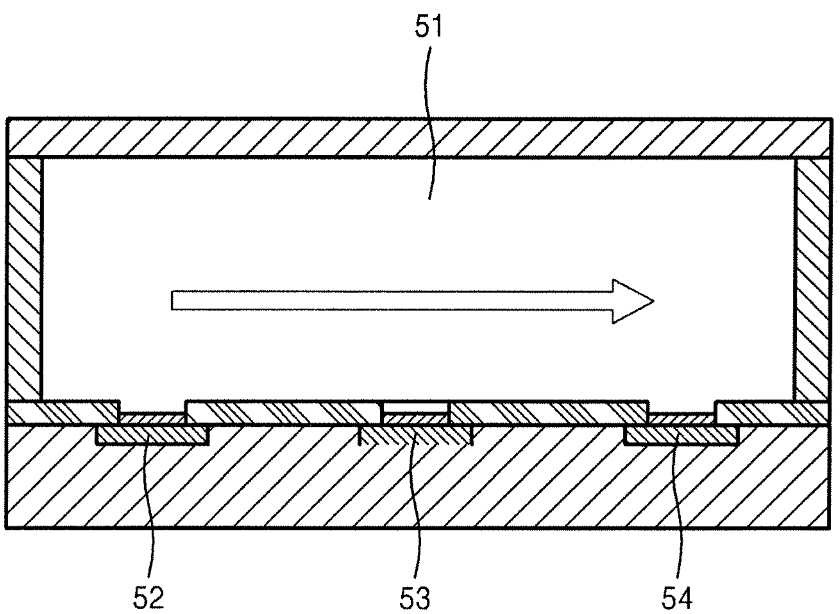

FIG. 6

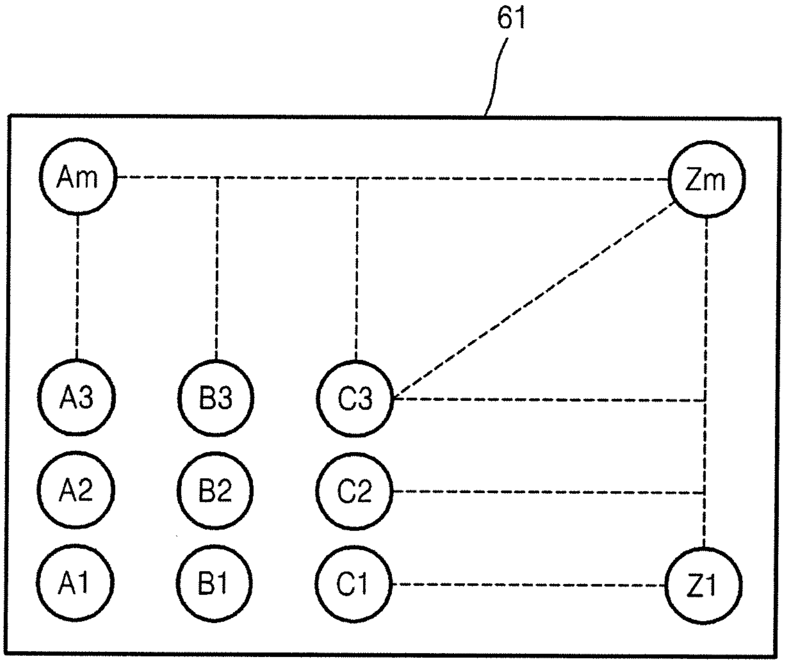

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4238757 A


### Non-patent literature cited in the description


- THE HOW AND WHY OF ELECTRONIC NOSES. IEEE SPECTRUM. IEEE INC, September 1998, vol. 35, 22-34
- Electronic noses - development and future prospects. **CRAVEN M A et al.** TRAC, TRENDS IN ANALYTICAL CHEMISTRY. ELSEVIER, October 1996, vol. 15, 486-493
- DETECTION OF VAPOURS AND ODOURS FROM A MULTISENSOR ARRAY USING PATTERN-RECOGNITION TECHNIQUES. OPART 2. ARTIFICIAL NEURAL NETWORKS. **GARDNER J W et al.** SENSORS AND ACTUATORS B. ELSEVIER SEQUOIA S.A, 01 July 1992, vol. B09, 9-15
- SIGNALVERARBEITUNG BEI CHEMOSENSOR-ARRAYS. **HORNER G.** TECHNISCHES MESSEN TM. R.OLDENBOURG VERLAG, 01 April 1995, vol. 62, 166-172
- Electronic noses: a review of signal processing techniques. **HINES E L et al.** IEE PROCEEDINGS: CIRCUITS DEVICES AND SYSTEMS. INSTITUTION OF ELECTRICAL ENGINEERS, 06 December 1999, vol. 146, 297-310